(19) Europäisches Patentamt
European Patent Office
Office européen des brevets

(11) Veröffentlichungsnummer: **0 185 964**
**B1**

(12) # EUROPÄISCHE PATENTSCHRIFT

(45) Veröffentlichungstag der Patentschrift: **28.02.90**

(21) Anmeldenummer: **85115209.0**

(22) Anmeldetag: **30.11.85**

(51) Int. Cl.⁵: **C 07 D 401/12**, C 07 D 401/14, C 07 D 409/14, C 07 D 405/14, C 07 D 413/14, C 07 D 491/048, A 61 K 31/50 // C07D237/04, C07D237/10

(54) **Dihydropyridin-carbonsäureamide, Verfahren zu ihrer Herstellung und ihre Verwendung in Arzneimitteln.**

(30) Priorität: **15.12.84 DE 3445852**

(43) Veröffentlichungstag der Anmeldung: **02.07.86 Patentblatt 86/27**

(45) Bekanntmachung des Hinweises auf die Patenterteilung: **28.02.90 Patentblatt 90/09**

(84) Bennante Vertragsstaaten: **AT BE CH DE FR GB IT LI NL SE**

(56) Entgegenhaltungen:
**EP-A-0 002 208**
**EP-A-0 107 735**
**EP-A-0 127 826**

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Meyer, Horst, Dr.**
**Henselweg 11**
**D-5600 Wuppertal 1 (DE)**
Erfinder: **Franckowiak, Gerhard, Dr.**
**Henselweg 10**
**D-5600 Wuppertal 1 (DE)**
Erfinder: **Rosentreter, Ulrich, Dr.**
**Kondorweg 22**
**D-5600 Wuppertal 1 (DE)**
Erfinder: **Gross, Rainer, Dr.**
**Platzhofstrasse 23**
**D-5600 Wuppertal 1 (DE)**
Erfinder: **Thomas, Günter, Dr.**
**Henselweg 5**
**D-5600 Wuppertal 1 (DE)**
Erfinder: **Schramm, Matthias, Dr.**
**Paffrather Strasse 38**
**D-5000 Köln 80 (DE)**
Erfinder: **Kayser, Michael, Dr.**
**Fleyerstrasse 231**
**D-5800 Hagen 1 (DE)**
Erfinder: **Seuter, Friedel, Dr.**
**Moospfad 16**
**D-5600 Wuppertal 1 (DE)**
Erfinder: **Perzborn, Elisabeth, Dr.**
**Am Teescherbusch 13**
**D-5600 Wuppertal 11 (DE)**
Erfinder: **Bechem, Martin, Dr.**
**Obere Bergerheide 4**
**D-5600 Wuppertal 1 (DE)**

**EP 0 185 964 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die vorliegende Erfindung betrifft 1,4-Dihydropyridincarbonsäureamide, Verfahren zu ihrer Herstellung sowie ihre Verwendung in Arzneimitteln, insbesondere zur Bekämpfung von Kreislauferkrankungen und Thrombosen.

Aus EP-A-0 107 735, EP-A-0 127 826 und EP-A-0 002 208 sind bereits Dihydropyridinderivate bekannt, die sich in ihren Wirkungen jedoch eindeutig von den erfindungsgemäßen Verbindungen unterscheiden. Neben den teilweise bekannten blutdrucksenkenden Wirkungen und der thrombozytenaggregationshemmenden Wirkung zeigen die erfindungsgemäßen Verbindungen eine ausgeprägte positiv inotrope Wirkung bei niedriger Dosierung. Sie besitzen somit ein neues Wirkprofil, das bei Kenntnis des Standes der Technik nicht erwartet werden konnte.

Die Erfindung betrifft 1,4-Dihydropyridincarbonsäureamide der allgemeinen Formel (I)

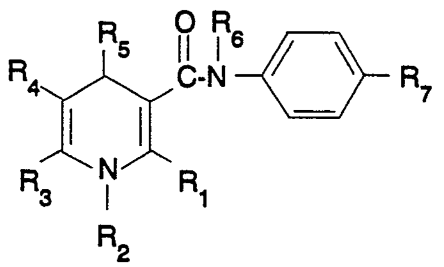

(I)

in welcher

$R_1$, $R_3$ gleich oder verschieden sind und für Cyano, für gegebenenfalls durch ein oder mehrere Fluor, Chlor, Brom, $C_1$-$C_2$-Alkoxycarbonyl, Acetyloxy, Pyridyl oder Hydroxy substituiertes Methyl oder Ethyl stehen,

$R_2$ für Wasserstoff, Methyl oder Ethyl steht,

$R_4$ für Wasserstoff, Nitro, Cyano oder für den Rest $COR^8$, $CO_2R^8$ oder $SO_2R^8$ steht,

wobei

$R^8$ für geradkettiges, verzweigtes oder cyclisches Alkyl oder Alkenyl mit bis zu 6 C-Atomen steht, das gegebenenfalls substituiert ist durch ein oder mehrere Fluor, Nitro, Cyano, $C_1$-$C_3$-Alkoxy, $C_1$-$C_3$-Alkylthio, Methylbenzylamino, gegebenenfalls durch Methoxy substituiertes Phenyl, Pyridyl oder Trifluormethyl

oder wobei $R^8$ eine direkte Bindung zu $R^3$ darstellt (für $R^3$ = Cyano),

$R^5$ für Phenyl oder Naphthyl steht, das gegebenenfalls 1 – 2 gleiche oder verschiedene Substituenten tragen kann, wobei als Substituenten Methyl, Trifluormethyl, Nitro, Cyano, $C_1$-$C_4$-alkoxy, $C_1$-$C_4$-Alkylthio, gelten, wobei Alkoxy und Alkylthio wiederum durch ein oder mehrere Fluor oder Phenyl substituiert sein können,

für den Rest

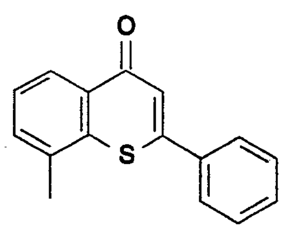

oder

für Pentafluorphenyl, Thienyl, Furyl, Pyridyl oder Benzoxadiazolyl steht,
für geradkettiges oder verzweigtes, gegebenenfalls durch Pyridyl substituiertes $C_1$-$C_6$-Alkyl steht, oder
für Cyclopentyl oder Cyclohexyl steht,

$R^6$ für Wasserstoff oder für geradkettiges oder verzweigtes $C_1$-$C_3$-Alkyl steht,

$R_7$ für die Gruppe

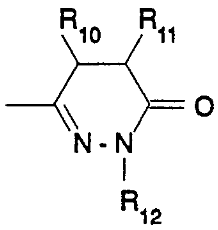 oder 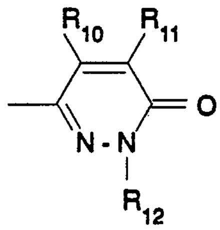

wobei

$R_{10}$, $R_{11}$ gleich oder verschieden sein können und für geradkettiges oder verzweigtes $C_1$-$C_4$-Alkyl stehen

$R_{12}$ die gleiche Bedeutung hat wie $R_6$ und wobei $R_6$ und $R_{12}$ gleich oder verschieden sein können,

in Form von Isomeren, Isomerengemischen, Racematen und optischen Antipoden sowie deren physiologisch unbedenklichen Salze.

Die erfindungsgemäßen Verbindungen können in Form ihrer Satze vorliegen. Im allgemeinen sind dies Salze mit anorganischen oder organischen Säuren. Bevorzugt sind jedoch die physiologisch unbedenklichen Salze der erfindungsgemäßen Stoffe mit anorganischen und organischen Säuren. Als Beispiele seien genannt: Hydrohalogenide, Hydrogensulfate, Sulfate, Hydrogenphosphate, Acetate, Maleate, Citrate, Furmarate, Tartrate, Lactate oder Benzoate.

Die erfindungsgemäßen Verbindungen sind neu und besitzen wertvolle pharmakologische Eigenschaften. Sie sind gefäß-dilatierend und positiv inotrop. Aufgrund dieser Eigenschaften können sie zur Bekämpfung von Kreislauferkrankungen eingesetzt werden und stellen somit eine Bereicherung der Pharmazie dar.

Die erfindungsgemäßen Verbindungen der allgemeinen Formel (I), in welcher

$R^1$-$R^{12}$ die oben angegebene Bedeutung haben,

$R^8$ jedoch keine direkte Bindung zu $R^3$ ist,

erhält man, wenn man

[A] Aldehyde der allgemeinen Formel (II)

$R^5$–C=O
H (II)

in welcher
$R^5$ die oben angegebene Bedeutung hat, mit Ketoverbindungen der allgemeinen Formel (III)

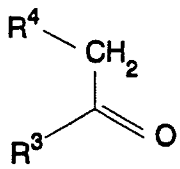 (III)

in welcher $R^3$, $R^4$ die angegebene Bedeutung haben,
oder deren Kondensationsprodukte der allgemeinen Formel (IV)

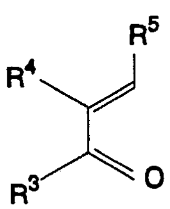 (IV)

in welcher $R^3$, $R^4$, $R^5$ die oben angegebene Bedeutung haben,
mit Enaminen der allgemeinen Formel (V)

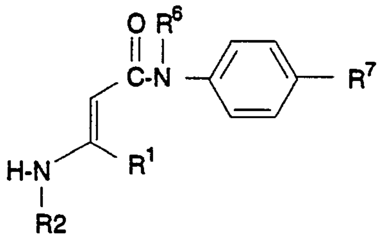

(V)

in welcher $R^1$, $R^2$, $R^6$, $R^7$ die oben angegebene Bedeutung haben, umsetzt, oder

[B] Aldehyde der allgemeinen Formel (II) und Ketoverbindungen der allgemeinen Formel (VI)

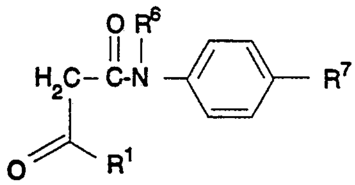

(VI)

in welcher $R^1$, $R^6$, $R^7$ die oben angegebene Bedeutung haben, oder deren Kondensationsprodukte der allgemeinen Formel (VII),

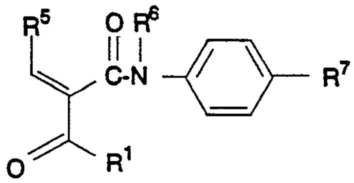

(VII)

in welcher $R^1$, $R^5$, $R^6$, $R^7$ die oben angegebene Bedeutung haben, mit Enaminen der allgemeinen Formel (VIII)

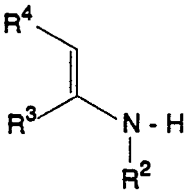

(VIII)

in welcher $R^2$, $R^3$, $R^4$ die oben angegebene Bedeutung haben, umgesetzt, oder wenn man

[C] 1,3-Dihydropyridin-carbonsäuren der allgemeinen Formel (IX)

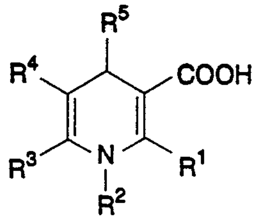

(IX)

in welcher $R^1$-$R^5$ die oben angegebene Bedeutung haben, nach bekannten Methoden, gegebenenfalls über ein reaktives Säurederivat, mit Verbindungen der allgemeinen Formel (X)

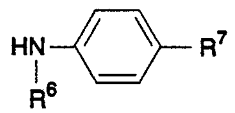

(X)

in welcher $R^6$, $R^7$ die oben angegebene Bedeutung haben, gegebenenfalls in einem inerten organischen Lösungsmittel umsetzt,
oder zur Herstellung derjenigen Verbindungen, in denen $R_4$ für die Gruppe $CO_2R_8$ steht und $R_8$ eine Bindung zu $R_3$ aufweist,

[D] Verbindungen der allgemeinen Formel Ia

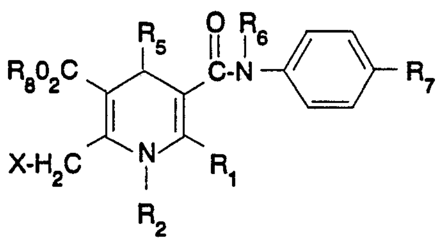

(Ia)

in welcher
$R_1$, $R_2$, $R_5$-$R_7$ die oben angegebene Bedeutung,

$R_8$ die oben angegebene Bedeutung hat aber keine direkte Bindung bedeutet und
X für Halogen steht,

pyrolisiert oder falls X für O-Acetyl oder O-Benzyl steht, gegebenenfalls in Anwesenheit von Basen cyclisiert.

Als reaktive Säurederivate seien beispielhaft genannt:
aktivierte Ester, Hydroxysuccinimidester, Säureimidazolide, gemischte Anhydride, Umsetzung mit Dicyclohexylcarbodiimid.

Setzt man beispielsweise nach Verfahrensvariante [A] als Ausgangsstoffe 3-Methoxybenzaldehyd, Acetessigsäuremethylester, oder das Kondensationsprodukt 3-Methoxybenzylidenacetessigsäuremethylester, und 3-Aminocrotonsäure-N-[4-(6-oxo -1,6-dihydropyridazin-3-yl)phenyl]amid ein, so läßt sich die Reaktion durch folgendes Schema darstellen:

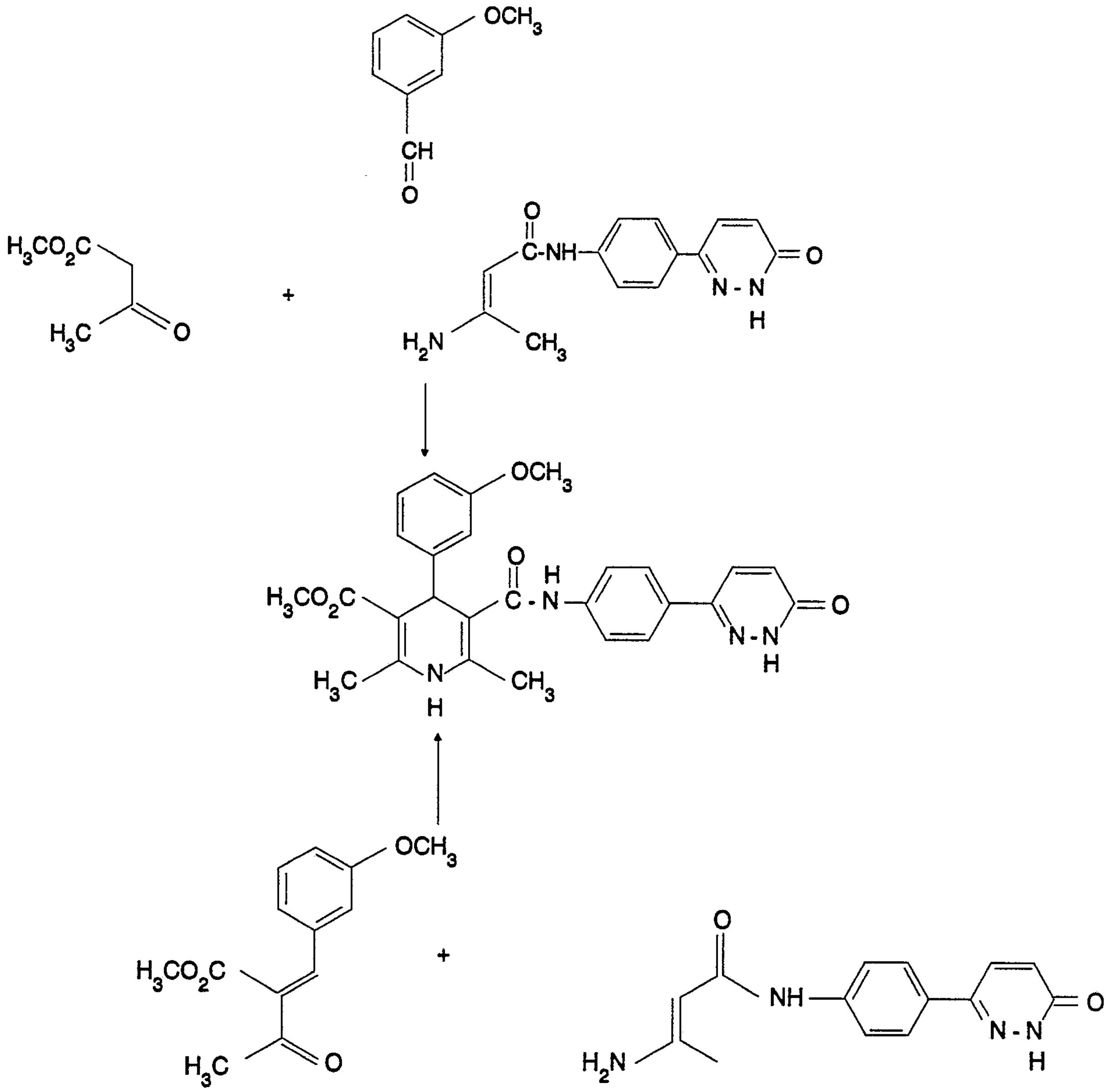

Setzt man nach Verfahrensvariante [B] als Ausgangsstoffe 2-Trifluormethylbenzaldehyd, Acetessigsäure-N-[4-(6-oxo-1,4,5,6-tetrahydropyridazin-3-yl)phenyl]amid oder das Kondensationsprodukt 2-Trifluormethyl-benzylidenacetessigsäure-N-[4-(6-oxo-1,4,5,6-tetrahydropyridazin-3-yl)phenyl]amid und 3-Aminocrotonsäureethylester ein, so läßt sich die Reaktion durch folgendes Schema darstellen:

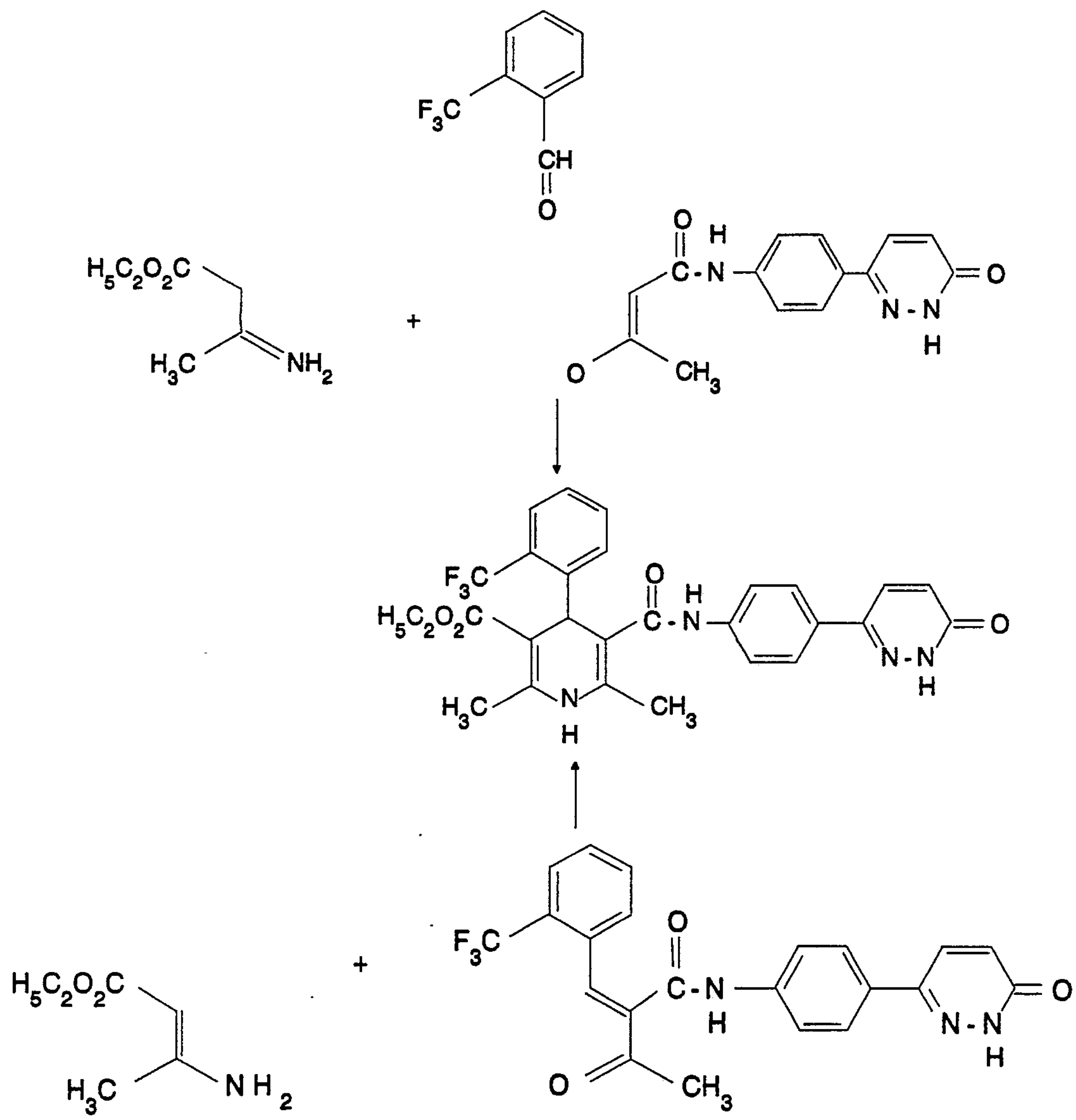

Setzt man nach Verfahrensvariante [C] 1,4-Dihydro-2,6-dimethyl-5-nitro-4-(3-nitrophenyl)-3-carbonsäure mit Carbonyldiimidazol um und läßt das erhaltene Imidazolid mit 4-(6-oxo-1,4,5,6-tetrahydropyridazin-3-yl)anilin reagieren, so läßt sich die Reaktion durch folgendes Schema darstellen:

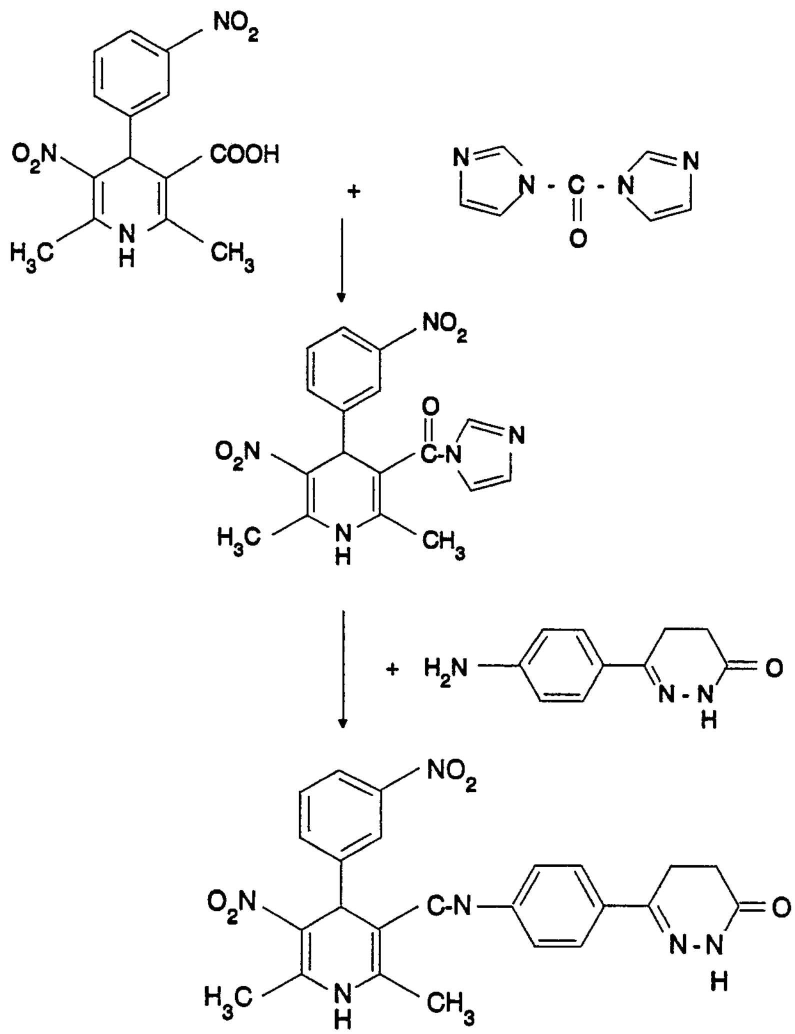

Die als Ausgangsstoffe verwendeten Verbindungen der Formeln II – IV, VIII – X sind literaturbekannt oder können nach literaturbekannten Methoden hergestellt werden (vgl. D. Borrmann "Umsetzung von Diketen mit Alkoholen, Phenolen und Mercaptanen" in Houben-Weyl, Methoden der organischen Chemie, Band VII/4, 230 ff. (1968); G. Jones, "The Knoevenagel Condensation" in Org. Reactions, Vol. VI, 204 ff (1967); SA. Glickman, A. C. Cope, J. Am. Chem. Soc. *67,* 1017 (1945); Deutsche Offenlegungsschriften 2 165 260, 2 847 237 und 2 401 665; A. Dornow und W. Sassenberg, Liebigs Ann. Chem. *602,* 14 (1957); EP-OS-71 819).

Die Verbindungen V, VI und VII sind neu. Sie können nach literaturbekannten Methoden, wie in den Beispielen beschrieben, hergestellt werden. Beispielsweise ebenfalls nach: D. Borrmann in Houben-Weyl, Methoden der organischen Chemie, Band VII/4, 230 ff (1968); G. Jones in Organic Reactions, Vol. VI, 204 ff (1967); S. A. Glickmann A. C. Cope in J. Amer. Chem. Soc. *67,* 1017 (1945).

Als Verdünnungsmittel kommen für Verfahren A und B alle inerten organischen Lösungsmittel in Frage. Hierzu gehören bevorzugt Alkohole wie Methanol, Ethanol, Propanol, Isopropanol, Ether wie Diethylether, Dioxan, Tetrahydrofuran oder Glykolmonoethylether, Eisessig, Pyridin, Dimethylformamid, Acetonitril, Dimethylsulfoxid oder Hexamethylphosphorsäuretriamid.

Für Verfahren C kommen die üblichen inerten organischen Lösungsmittel in Frage. Hierzu gehören bevorzugt chlorierte Kohlenwasserstoffe wie Dichlormethan, Trichlormethan, Tetrachlormethan oder 1,2-Dichlorethan, Ether wie Diethylether, Tetrahydrofuran, Dioxan oder 1,2-Dimethoxyethan, aromatische Kohlenwasserstoffe wie Toluol oder Xylol, Acetonitril, Nitromethan, Dimethylformamid, Hexamethylphosphorsäuretriamid, Pyridin, Essigsäureethylester.

Die Reaktionstemperaturen für alle Verfahren können in einem größeren Bereich variiert werden. Im allgemeinen arbeitet man bei Verfahren A und B in einem Bereich von 10°C bis 200°C, bevorzugt von 20°C bis 150°C. Bei Verfahren C arbeitet man im allgemeinen in einem Bereich von –70°C bis +60°C, bevorzugt von –50°C bis +40°C.

Die Umsetzung kann bei Normaldruck, aber auch bei erhöhtem oder verringertem Druck durchgeführt werden. Im allgemeinen arbeitet man bei Normaldruck.

Bei der Durchführung der erfindungsgemäßen Verfahren ist das Verhältnis der an der Reaktion beteiligten

Stoffe beliebig. Im allgemeinen arbeitet man jedoch mit molaren Mengen der Reaktanden. Bei Verfahren C hat es sich als zweckmäßig erwiesen, das Amin im bis zu 5-fach molaren Überschuß einzusetzen.

Erfindungsgemäße Verbindungen der allgemeinen Formel (I) in welcher

$R^1$-$R^3$, $R^5$-$R^{12}$ die oben angegebene Bedeutung haben,
$R^4$ für die Gruppe $CO_2R^8$ steht und
$R^8$ für eine direkte Bindung zu $R^3$ steht

erhält man, wenn man Verbindungen der allgemeinen Formel (Ia)

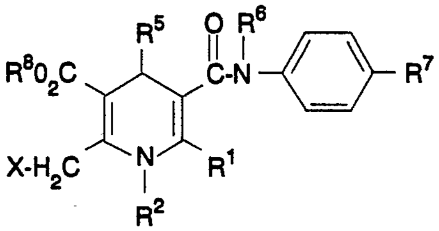

(Ia)

in welcher

$R^1$, $R^2$, $R^5$-$R^7$ die oben angegebene Bedeutung haben,
$R^8$ keine direkte Bindung bedeutet und
X für Halogen, bevorzugt Chlor oder Brom steht

mit oder ohne Lösungsmittel pyrolysiert, oder falls X für O-Acetyl oder O-Benzyl steht, gegebenenfalls in Anwesenheit von Basen cyclisiert.

Die Pyrolyse kann mit oder ohne Lösungsmittel durchgeführt werden. Als Lösungsmittel kommen gegebenenfalls alle üblichen inerten organischen Lösungsmittel in Frage. Dazu zählen aromatische Kohlenwasserstoffe wie Benzol, Toluol oder Xylol, Tetralin, Erdölfraktionen, Ether wie Diethylether, Dioxan, Tetrahydrofuran, Glykolmono- bzw. diethylether, Halogenkohlenwasserstoffe wie Di-, Tri- oder Tetrachlormethan, Dichlor- oder Trichlorethylen.

Die Pyrolyse wird in einem Temperaturbereich von 20°C bis 300°C, bevorzugt von 40°C bis 250°C durchgeführt.

Die Pyrolyse kann bei normalem, erhöhtem oder erniedrigten Druck durchgeführt werden. Im allgemeinen arbeitet man bei Normaldruck.

Als Basen eignen sich die üblichen Basen wie zum Beispiel Alkali- oder Erdalkalihydroxide, besonders Natrium-, Kalium-, Calciumhydroxid oder Amine wie Ammoniak, Triethylamin, Pyridin. Die Cyclisierung kann in den üblichen Lösungsmitteln wie aromatische Kohlenwasserstoffe (z. B. Benzol, Toluol) Alkoholen (Ethanol, Propanol, Methanol) oder Essigsäure durchgeführt werden. Die Cyclisierung erfolgt bei Temperaturen von 0°C bis 200°C, bevorzugt bei 20–150°C.

Verwendet man als Ausgangsstoff 2-Brommethyl-1,4-dihydro-6-methyl-4-(2-trifluormethylphenyl)pyridin-3,5-dicarbonsäure-3-methylester-5-N-[4-(6-oxo-1,4,5,6-tetrahydropyridazin-3-yl)phenyl]amid, so läßt sich die Reaktion durch folgendes Schema verdeutlichen:

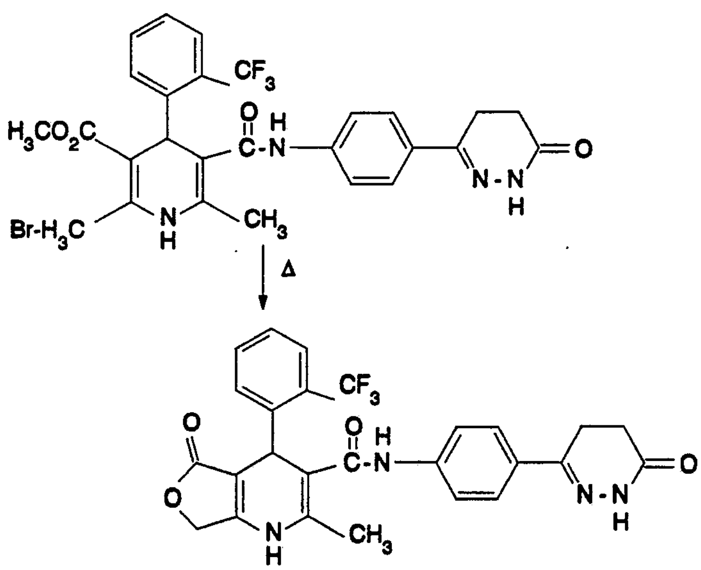

Die vorstehenden Herstellungsverfahren sind lediglich zur Verdeutlichung angegeben. Die Herstellung der Verbindungen der Formel (I) ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie Diastereomerengemische. Die Racemformen lassen sich ebenso wie die Diastereomeren in bekannter Weise in die stereoisomer einheitlichen Bestandteile trennen (vgl. E. L. Eliel, Stereochemistry of Carbon Compounds, McGraw Hill, 1962).

Die erfindungsgemäßen Verbindungen haben eine positiv inotrope und koronardilatierende Wirkung und zeigen somit ein nicht vorhersehbares und wertvolles pharmakologisches Wirkspektrum. Sie können als kreislaufbeeinflussende Mittel, als Koronartherapeutika, Antiarrhythmika, zur Behandlung von Herzinsuffizienz und zur Beeinflussung des Blutzuckerspiegels eingesetzt werden. Darüber hinaus wirken die neuen Stoffe thrombozytenaggregationshemmend und eignen sich damit zur Behandlung von Thrombosen, Thromboembolien sowie Ischämien.

Die inotropen und koronardilatierenden Wirkungen werden am isoliert perfundierten Herzen des Meerschweinchens gefunden, wobei besonders solche Verbindungen bevorzugt sein sollen, die neben einer die Kontraktilität des isolierten Herzens steigernden eine den Perfusionsdruck senkend und damit die Koronarien dilatierende Wirkung aufweisen.

Dazu werden die Herzen von 250 bis 350 g schweren Albino Meerschweinchen verwendet. Die Tiere werden mit einem Schlag auf den Kopf getötet, der Thorax geöffnet, in die freipräparierte Aorta eine Metallkanüle eingebunden und der linke Vorhof geöffnet. Das Herz wird mit den Lungen aus dem Thorax herausgetrennt und über die Aortenkanüle an die Perfusionsapparatur bei laufender Perfusion angeschlossen. Die Lungen werden an den Lungenwurzeln abgetrennt. Als Perfusionsmedium dient Krebs-Henseleit-Lösung (2) (118,5 mmol/l NaCl, 4,75 mmol/l KCl, 1,19 mmol/l $KH_2PO_4$, 1,19 mmol/l $MgSO_4$, 25 mmol/l $NaHCO_3$, 0,013 mmol/l NaEDTA), deren $CaCl_2$ je nach Bedarf variiert wird, in der Regel jedoch 1,2 mmol/l beträgt. Als energieliefernes Substrat werden 10 mmol/l Glucose zugesetzt. Vor der Perfusion wird die Lösung partikelfrei filtriert. Die Lösung wird mit Carbogen (95 % $O_2$, 5 % $CO_2$ zur Aufrechterhaltung des pH-Werten von 7,4) begast. Die Herzen werden mit konstantem Fluß (10 ml/min) bei 32°C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt und die isovolumetrischen Kontraktionen auf einem Schnellschreiber registriert. (Ope, L., J. Physiol. *180* (1965) 529 - 541). Der Perfusionsdruck wird mittels eines Druckaufnehmers, der vor dem Herzen mit dem Perfusionssystem in Verbindung steht, registriert. Unter diesen Bedingungen zeigt eine Senkung des Perfusionsdrucks eine Koronardilatation, eine Steigerung der links ventrikulären Druckamplitude einen Anstieg der Herzkontraktilität an.

Die erfindungsgemäßen Verbindungen werden in geeigneten Verdünnungen in das Perfusionssystem kurz vor den isolierten Herzen infundiert.

In der folgenden Tabelle sind die kontraktilitätssteigernden und koronardilatierenden Effekte am isoliert perfundierten Meerschweinchenherzen von einigen Beispielen aufgeführt.

**Tabelle:**

Isoliert perfundiertes Meerschweinchenherz. Kontraktionsamplitude-steigernder und Perfusionsdrucksenkender Effekt einiger erfindungsgemäßer Verbindungen (prozentuale Änderung gegenüber Kontrollbedingungen).

| Beispiel-Nr. | Konzentration (g/ml) $10^{-7}$ | | $10^{-6}$ | |
|---|---|---|---|---|
| | KA | PD | KA | PD |
| 2 | +12 | − 7 | +101 | −41 |
| 3 | +44 | −18 | + 52 | −9 |
| 6 | +43 | 19 | + 67 | −40 |

KA = Kontraktionsamplitude
PD = Perfusionsdruck

Die thrombozytenaggregationshemmende Wirkung wurde in folgenden Versuchsanordnungen gefunden:

in Plasma

Für die in vitro Versuche wurde Blut von gesunden Probanden beiderlei Geschlechts verwendet. Als Antikoagulans wurden einem Teil 3,8 %-iger wäßriger Natriumzitratlösung 9 Teile Blut zugemischt. Mittels Zentrifugation erhält man aus diesem Blut plättchenreiches Zitratplasma (PRP) (Literatur: Jürgens/Beller, Klinische Methode der Blutgerinnungsanalyse; Thieme Verlag, Stuttgart 1959).

Für diese Untersuchungen wurden 0,8 ml PRP und 0,1 ml der Wirkstofflösung bei 37°C im Wasserbad

vorinkubiert. Anschließend wurde die Thrombozytenaggregation nach der turbidometrischen Methode (Literatur: Born, B.V.R., J. Physiol. (London), *162*, 67, 1962) im Aggregometer bei 37°C bestimmt (Literatur: Therapeutische Berichte 47, 80 - 86, 1975). Hierzu wurde die vorinkubierte Probe mit 0,1 ml Kollagen, einem aggregationsauslösenden Agens, versetzt. Die Veränderung der optischen Dichte in der Probe des PRP wurde während einer Zeitdauer von 6 Minuten aufgezeichnet und der Ausschlag nach 6 Minuten bestimmt. Hierzu wird die prozentuale Hemmung gegenüber der Kontrolle errechnet.

| Beispiel-Nr. | Grenzkonzentration für Hemmung (mg/l) |
|---|---|
| 3 | 0,01 – 0,003 |
| 5 | 0,1 – 0,03 |
| 6 | 0,3 – 0,1 |
| 7 | 3 – 1 |
| 9 | 3 – 1 |
| 10 | 0,1 – 0,03 |
| 11 | 0,03 – 0,01 |
| 12 | 0,03 – 0,01 |
| 13 | 0,03 – 0,01 |
| 24 | 0,03 – 0,01 |
| 55 | 1 – 0,3 |
| 56 | 0,3 – 0,1 |

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:
Wasser, nicht-toxische organische Lösungsmittel, wie Paraffine (z. B. Erdölfraktionen), pflanzliche Öle (z. B. Erdnuß-/Sesamöl), Alkohole (z. B. Ethylalkohol, Glycerin), Glykole (z. B. Propylenglykol, Polyethylenglykol), feste Trägerstoffe, wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Rohr-, Milch- und Traubenzucker), Emulgiermittel (z. B. Polyoxyethylen-Fettsäure-Ester, Polyoxyethylen-Fettalkohol-Ether, Alkylsulfonate und Arylsulfonate), Dispergiermittel (z. B. Lignin, Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffe, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengenannten Hilfsstoffen mit verschiedenen Geschmacksaufbesseren oder Farbstoffe versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körnergewicht des Versuchstieres bzw. der Art des Applikationsweges, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

**Beispiel 1**

4- ( 2-Benzylmercaptophenyl ) -1,4-dihydro-2,6-dimethyl-5 -nitro-pyridin-3-carbonsäure[4 (6-oxo-1,4,5,6-tetrahydropyridazin-3-yl)phenyl]amid

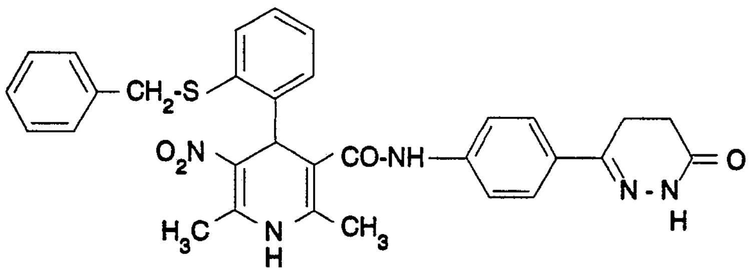

a) Acetessigsäure-N- [4-(6-oxo-1,4,5,6-tetrahydropyridazin-3-yl)phenyl] amid

20 g (0,106 mol) 3-(4-Aminophenyl)-6-oxo-1,4,5,6-tetrahydropyridazin werden in 250 ml Dimethylformamid mit 9,47 g (0,113 mol) Diketen bei Raumtemperatur 12 h gerührt. Nach Einengen wird mit 100 ml Ethanol aufgekocht und nach dem Abkühlen abfiltriert.
Ausbeute: 79,2 % der Theorie
Schmelzpunkt: 206°C

b) 3-Aminocrotonsäure-N-[ 4-(6-oxo-1,4,5,6-tetrahydropyridazin-3-yl)phenyl] amid

12,5 g (45,7 mmol) Acetessigsäure-N-[4-(6-oxo-1,4,5,6-tetrahydropyridazin-3-yl)phenyl]amid werden in 225 ml wäßrigem konzentriertem Ammoniak 2 h am Rückfluß erhitzt. Nach dem Abkühlen wird abgesaugt und mit Wasser neutral gewaschen.
Ausbeute: 87 % der Theorie
Schmelzpunkt: 237°C

c) 4-(2-Benzylmercaptophenyl )-1,4-dihydro-2,6-dimethyl-5-nitro-pyridin-3-carbonsäure-N-[4-(6-oxo-1,4,5,6 -tetrahydropyridazin-3-yl)phenyl] amid

10 mmol 2-Benzylmercaptobenzaldehyd, 15 mmol Nitroaceton und 10 mmol 3-Aminocrotonsäure-N-[4-(6-oxo-1,4,5,6-tetrahydropyridazin-3-yl )-phenyl]amid werden in 30 ml Isopropanol 10 h auf 60 – 70°C erhitzt. Die Reaktionsmischung wird nach Einengen und Aufnehmen in Chloroform an Kieselgel mit Chloroform unter Zugabe von Methanol chromatografiert. Das Produkt wird aus Isopropanol umkristallisiert.
Ausbeute: 32 % der Theorie
Schmelzpunkt: 240 – 242°C (Z)

**Beispiel 2**

1,4-Dihydro-2,6-dimethyl-5-nitro-4(2-trifluormethylphenyl )-pyridin-3-carbonsäure-N-[4-( 6-oxo-1,4,5,6-tetrahydropyridazin-3-yl )phenyl]amid

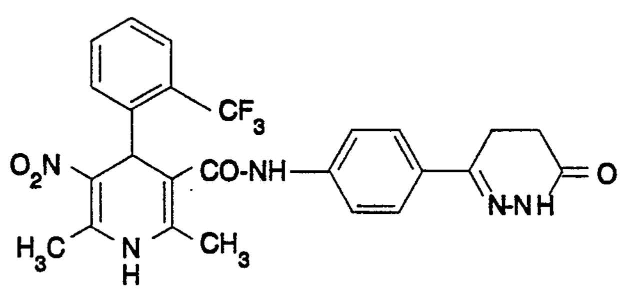

10 mmol 2-Nitro-1-(2-trifluormethylphenyl)-buten-1-on-3 und 10 mmol 3-Aminocrotonsäure-N-[4-(6-oxo-1,4,5,6-tetrahydropyridazin-3-yl)phenyl]amid werden in 30 ml Isopropanol 8 h auf 60 – 70°C erhitzt. Das Produkt kristallisiert aus der warmen Reaktionsmischung aus und wird durch Umkristallisation aus Chloroform gereinigt.
Ausbeute: 62 % der Theorie
Schmelzpunkt: 208°C

**Beispiel 3**

1,4-Dihydro-4-( 2-methoxyphenyl )-2,6-dimethyl-pyridin-3,5-dicarbonsäure-3-ethylester-5-N-[4-( 6-oxo-1,4,5,6-tetrahydropyridazin-3-yl) phenyl]amid

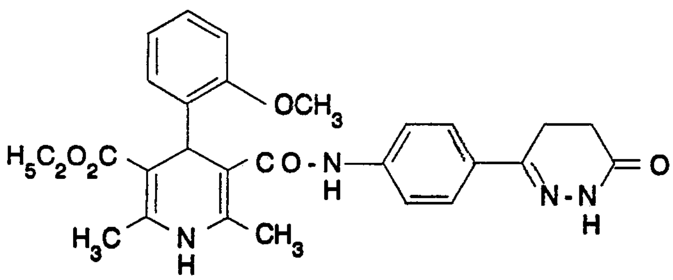

10 mmol 2-Methoxybenzylidenacetessigsäure-ethylester und 10 mmol 3-Aminocrotonsäure-N-[4-(6-oxo-1,4,5,6-tetrahydropyridazin-3-yl)-phenyl] werden in 20 ml Diglykol 6 h auf 120°C erhitzt. Nach dem Abkühlen wird auf Eis gegossen, der Niederschlag wird abgesaugt, getrocknet und aus Essigester umkristallisiert.
Ausbeute: 71 % der Theorie
Schmelzpunkt: 244°C

**Beispiel 4**

2-Methyl-4-( 3-nitrophenyl )-5-oxo-1,4,5,7-tetrahydrofuro -[3,5-b]pyridin-3-carbonsäure-N-[4-( 6-oxo-1,4,5,6-tetrahydropyridazin-3-yl)phenyl] amid

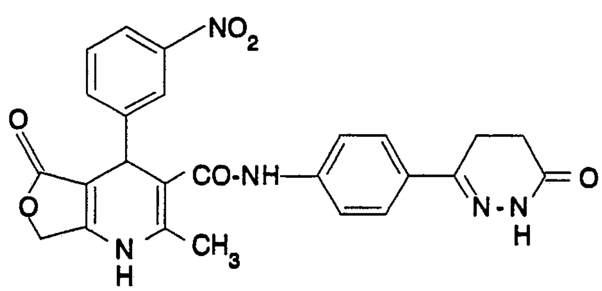

10 mmol 4-Chlor-2-ethoxycarbonyl-1-(3-nitrophenyl)-buten -1-on-3 und 10 mmol 3-Aminocrotonsäure-N-[4-(6-oxo-1,4,5,6 -tetrahydropyridazin-3-yl)phenyl]amid werden in 25 ml Eisessig 12 h auf 80°C erhitzt. Das Produkt kristallisiert beim Abkühlen aus und wird aus Eisessig umkristallisiert.
Ausbeute: 52 % der Theorie
Schmelzpunkt: 225 – 30°C (Z)

4-[3-Nitrophenylbenzyliden)acetoacetylamino]phenyl-4,5-dihydro-3-(2H)pyridazinon

10 mmol 3-Nitrobenzaldehyd und 10 mmol Acetessigsäure-N-[4-(6-oxo-1,4,5,6-tetrahydropyridazin-3-yl)phenyl]-amid werden mit 1 g Piperidinacetat in 150 ml Dimethylformamid auf 90°C erhitzt. Nach 12 h gießt man den Ansatz auf Eiswasser, filtriert den Niederschlag ab und reinigt ihn durch Auskochen mit Ethanol.
Ausbeute: 62 % der Theorie
Schmelzpunkt: 241°C

**Beispiel 5**

1,4-Dihydro-2,6-dimethyl-4-( 2-nitrophenyl )-pyridin-3,5-dicarbonsäure-3-ethylester-5-N-[4-( 6-oxo-1,4,5,6-tetrahydropyridazin-3-yl) phenyl]amid

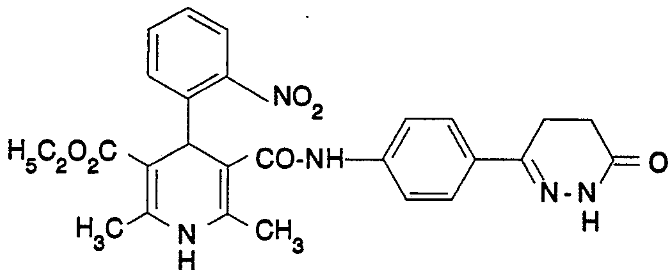

Zu einer Lösung von 10 mmol 1,4-Dihydro-2,6-dimethyl-4 -(2-nitrophenyl) -pyridin-3,5-dicarbonsäure-monoethylester in 50 ml absolutem Tetrahydrofuran gibt man bei Raumtemperatur 12 mmol Carbonyldiimidazol, die Lösung wird dann 30 Min. im Rückfluß erhitzt. Dann gibt man 10 mmol 3-Aminocrotonsäure-N-[4-(6-oxo-1,4,5,6-tetrahydropyridazin-3-yl)phenyl] amid und 50 mg Natriummethylat hinzu. Die Mischung wird 3 h im Rückfluß erhitzt, das Lösungsmittel im Vakuum abgedampft und der Rückstand aus Isopropanol umkristallisiert.
Ausbeute: 82 % der Theorie
Schmelzpunkt: 262°C

Die weiteren, in den nachfolgenden Tabellen aufgeführten Beispiele wurden analog den vorstehend beschriebenen Verfahren erhalten.

**Tabelle 1**

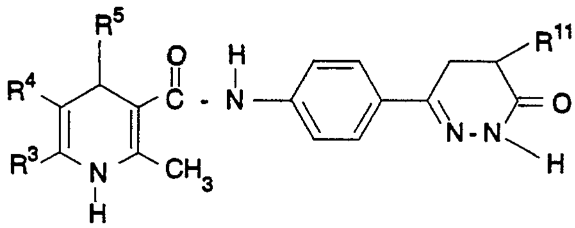

| Beispiel Nr. | $R^3$ | $R^4$ | $R^5$ | $R^{11}$ | Fp. [°C] |
|---|---|---|---|---|---|
| 6 | $CH_3$ | $CO_2C_2H_5$ | 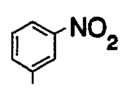 | H | 205 |
| 7 | $CH_3$ | $CO_2C_2H_5$ | 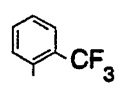 | H | 182 |
| 8 | $CH_3$ | $CO_2CH_3$ | 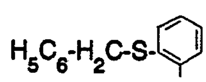 | H | 236 |
| 9 | $CH_3COOCH_2$ | $CO_2C_2H_5$ | 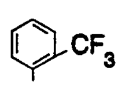 | H | 170 |
| 10 | $CH_3$ | $CO_2C_2H_5$ | 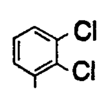 | H | 185 |
| 11 | $CH_3$ | $CO_2CH_3$ | 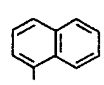 | H | 201 |

| Beispiel Nr. | $R^3$ | $R^4$ | $R^5$ | $R^{11}$ | Fp. [°C] |
|---|---|---|---|---|---|
| 12 | $CH_3$ | $CO_2C_2H_5$ | 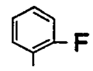 | H | 236 |
| 13 | $CH_3$ | $CO_2C_2H_5$ | 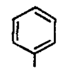 | H | 162 |
| 14 | $CH_3$ | $CO_2C_2H_5$ | 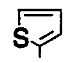 | H | |
| 15 | $CH_3$ | $COCH_3$ | 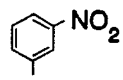 | H | |
| 16 | $CH_3$ | $CO_2C_2H_5$ | 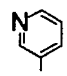 | H | |
| 17 | $CH_3$ | $CO_2C_2H_5$ | 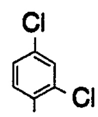 | H | 262 |
| 18 | $CH_3$ | $CO_2C_2H_5$ | 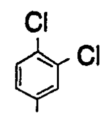 | H | 162 |
| 19 | $CH_3$ | $CO_2C_2H_5$ | 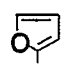 | H | |
| 20 | $CH_3$ | $CO_2C_2H_5$ | 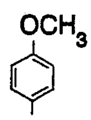 | H | |
| 21 | $CH_2CH_2CH_3$ | $CO_2C_2H_5$ | 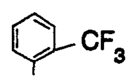 | H | 174 |
| 22 | $CH_3$ | $CO_2C_2H_5$ | 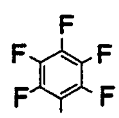 | H | 218 |
| 23 | $CH_3$ | $CO_2CH_3$ | 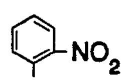 | H | 238 |
| 24 | $CH_3$ | $CO_2CH_3$ | 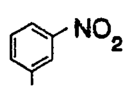 | H | 228 |
| 25 | $CH_3$ | $COCH_3$ | 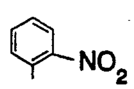 | H | 262 |

| Beispiel Nr. | $R^3$ | $R^4$ | $R^5$ | $R^{11}$ | Fp. [°C] |
|---|---|---|---|---|---|
| 26 | $CH_3$ | $CO_2CH_3$ | 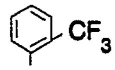 | H | 267 |
| 27 | $CH_3$ | $COCH_3$ | 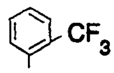 | H | 183 |
| 28 | $CH_3$ | $COCH_3$ | 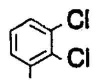 | H | 226 |
| 29 | $CH_3$ | $NO_2$ | 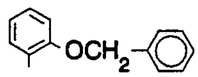 | H | 247 |
| 30 | $CH_3$ | $NO_2$ | 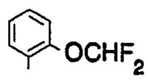 | H | 247 |
| 31 | $CH_3$ | $NO_2$ | 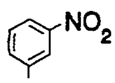 | H | 158 |
| 32 | $CH_3$ | $NO_2$ | 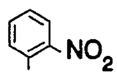 | H | 209 |
| 33 | $CH_3$ | $NO_2$ | 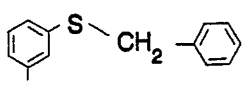 | H | 226 |
| 34 | $CH_3$ | $NO_2$ | 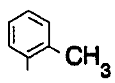 | H | 209 |
| 35 | $CH_3$ | $NO_2$ | 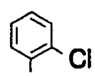 | H | 215 |
| 36 | $CH_3$ | $NO_2$ | 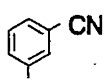 | H | 264 |
| 37 | $CH_3$ | $NO_2$ | 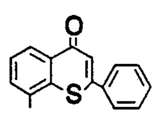 | H | 194 |
| 38 | $CH_3$ | $NO_2$ | 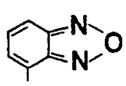 | H | 256 |
| 39 | $CH_3$ | $NO_2$ | 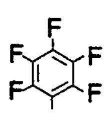 | H | 219 |
| 40 | $CH_3$ | $NO_2$ | 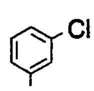 | H | 251 |

| Beispiel Nr. | $R^3$ | $R^4$ | $R^5$ | $R^{11}$ | Fp. [°C] |
|---|---|---|---|---|---|
| 41 | $CH_3$ | $NO_2$ | 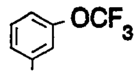 | H | 233 |
| 42 | $CH_3$ | $NO_2$ | 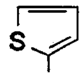 | H | 185 |
| 43 | $CH_3$ | $NO_2$ | $-(CH_2)_4-CH_3$ | H | 205 |
| 44 | $CH_3$ | H | 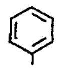 | H | 194 |
| 45 | $CH_3$ | $NO_2$ | 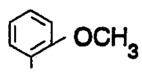 | H | 175 |
| 46 | $CH_3$ | $NO_2$ | 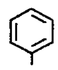 | H | 217 |
| 47 | $CH_3$ | $NO_2$ | 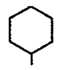 | H | 189 |
| 48 | $CH_3$ | $NO_2$ | 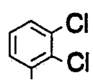 | H | 260 |
| 49 | $CH_3$ | $NO_2$ | $-CH_2-CH_3$ | H | 131 |
| 50 | $CH_3$ | $NO_2$ | 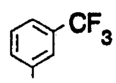 | H | 256 |
| 51 | $CH_3$ | $NO_2$ | 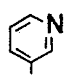 | H | 258 |
| 52 | $CH_3$ | $NO_2$ | 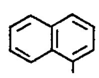 | H | 220 |
| 53 | $-CH_2$ ———— | $\overset{O}{\overset{\|}{OC}}-$ | 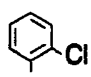 | H | 259 |
| 54 | $CH_3$ | $CO_2CH_3$ | $CH_2-CH_2-$ 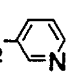 | H | 140–160 |
| 55 | $CH_3$ | $CO_2(CH_2)_3-$ 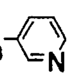 | 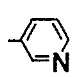 | H | 200 |
| 56 | $CH_3$ | $CO_2(CH_2)_3-$ 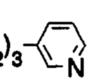 | $CH_2-CH_2-$ 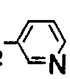 | H | 110–170 |
| 57 | $CH_3$ | $CO_2CH_2-$ 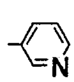 | $CH_2-CH_2-$ 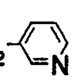 | H | 180 (Zers.) |

| Beispiel Nr. | $R^3$ | $R^4$ | $R^5$ | $R^{11}$ | Fp. [°C] |
|---|---|---|---|---|---|
| 58 | $CH_3$ | $CO_2C_3H_7$ | 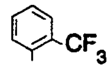 | H | 241 |
| 59 | $CH_3$ | $CH_3$<br>/<br>$CO_2CH$<br>\\<br>$CH_3$ | 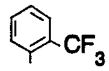 | H | 193 |
| 60 | $CH_3$ | $CO_2CH_2CH=CH_2$ | 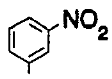 | H | |
| 61 | $CH_3$ | $CO_2CH_2C{\equiv}CH$ | 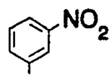 | H | |
| 62 | $CH_3$ | $SO_2CH_3$ | 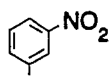 | H | 238 |
| 63 | $CH_3$ | $CO_2CH_3$ | 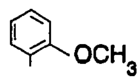 | H | 208 |
| 64 | $CH_3$ | $CO_2C_4H_9$–n | 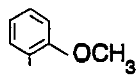 | H | 190 |
| 65 | $CH_3$ | $CO_2C_3H_7$–n | 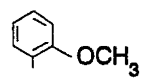 | H | 192 |
| 66 | $CH_3$ | $CO_2C_6H_{13}$–n | 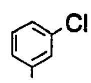 | H | 208 |
| 67 | $CH_3$ | $CO_2C_3H_7$–n | 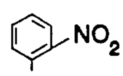 | H | 261 |
| 68 | $CH_3$ | $CO_2C_4H_9$–n | 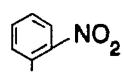 | H | 251 |
| 69 | $CH_3$ | $CO_2C_2H_4OCH_3$ | 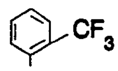 | H | 258 |
| 70 | $CH_3$ | $CO_2C_2H_5$ | 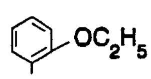 | H | 222 |
| 71 | $CH_3$ | $CO_2CH_3$ | 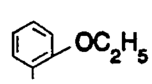 | H | 254 |
| 72 | $CH_3$ | $CO_2C_3H_7$–n | 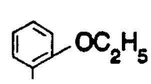 | H | 254 |
| 73 | $CH_3$ | $CO_2C_4H_9$–n | 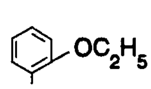 | H | 244 |
| 74 | $CH_3$ | $CO_2CH_3$ | 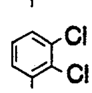 | H | 275 |
| 75 | $CH_3$ | $CO_2C_3H_7$–n | 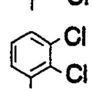 | H | 258 |

| Beispiel Nr. | $R^3$ | $R^4$ | $R^5$ | $R^{11}$ | Fp. [°C] |
|---|---|---|---|---|---|
| 76 | $CH_3$ | 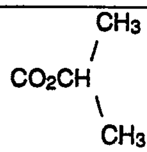 | 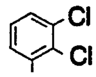 | H | 263 |
| 77 | $CH_3$ | $CO_2C_4H_9$–n | 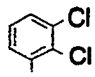 | H | 226 |
| 78 | $CH_3$ | $CO_2(CH_2)_2$- 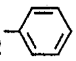 | 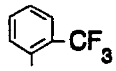 | H | 251 |
| 79 | $C_2H_7$ | $CO_2C_2H_5$ | 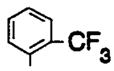 | H | 171 |
| 80 | $CH_3$ | $CO_2CH_2$- 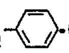 -$OCH_3$ | 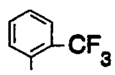 | H | 261 |
| 81 | $CH_3$ | $CO_2CH_2CH{=}CH_2$ | 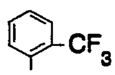 | H | 259 |
| 82 | $CH_3$ | $CO_2CH_2CH_2SCH_2CH_3$ | 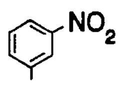 | H | 132 |
| 83 | $CH_3$ | $CO_2CH_3$ | 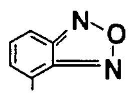 | H | 198 |
| 84 | $CH_3$ | $CO_2C_2H_5$ | 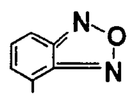 | H | 192 |
| 85 | CH3 | 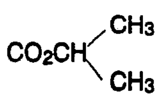 | 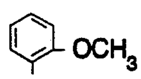 | H | 188 |
| 86 | $CH_3$ | 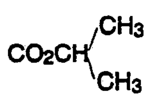 | 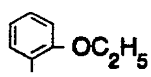 | H | 158 |
| 87 | $CH_3$ | $CO_2C_2H_5$ | 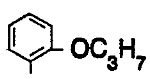 | H | 240 |
| 88 | $CH_3$ | $CO_2CH_3$ | 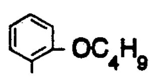 | H | 238 |
| 89 | $CH_3$ | $CO_2CH_2CH_2CN$ | 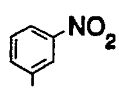 | H | 263 |

| Beispiel Nr. | $R^3$ | $R^4$ | $R^5$ | $R^{11}$ | Fp. [°C] |
|---|---|---|---|---|---|
| 90 | $CH_3$ | CN | 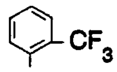 | H | 235 |
| 91 | $CH_3$ | $CO_2C_2H_5$ | 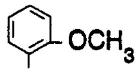 | $CH_3$ | 248 |
| 92 | $CH_3$ | $CO_2CH_3$ | 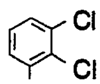 | $CH_3$ | 281 |
| 93 | $CH_3$ | $CO_2C_2H_5$ | 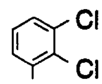 | $CH_3$ | 240 |
| 94 | $CH_3$ | $CO_2C_2H_5$ | 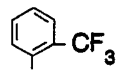 | $CH_3$ | 170 |
| 95 | $CH_3$ | $CO_2C_2H_5$ | 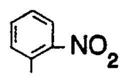 | $CH_3$ | 250 |
| 96 | $CH_3$ | $NO_2$ | 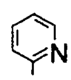 | H | 253 |

**Patentansprüche**

1. 1,4-Dihydropyridin-carbonsäureamide der allgemeinen Formel I

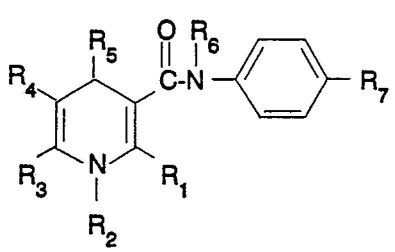

(I)

in welcher

$R_1$, $R_3$ gleich oder verschieden sind und für Cyano, für gegebenenfalls durch ein oder mehrere Fluor, Chlor, Brom, $C_1$-$C_2$-Alkoxycarbonyl, Acetyloxy, Pyridyl oder Hydroxy substituiertes Methyl oder Ethyl stehen,

$R_2$ für Wasserstoff, Methyl oder Ethyl steht,

$R_4$ für Wasserstoff, Nitro, Cyano oder für den Rest $COR^8$, $CO_2R^8$ oder $SO_2R^8$ steht,

wobei

$R^8$ für geradkettiges, verzweigtes oder cyclisches Alkyl oder Alkenyl mit bis zu 6 C-Atomen steht, das gegebenenfalls substituiert ist durch ein oder mehrere Fluor, Nitro, Cyano, $C_1$-$C_3$-Alkoxy, $C_1$-$C_3$-Alkylthio, Methylbenzylamino, gegebenenfalls durch Methoxy substituiertes Phenyl, Pyridyl oder Trifluormethyl oder wobei $R^8$ eine direkte Bindung zu $R^3$ darstellt (für $R^3 \neq$ Cyano),

$R^5$ für Phenyl oder Naphthyl steht, das gegebenenfalls 1 – 2 gleiche oder verschiedene Substituenten tragen kann, wobei als Substituenten Methyl, Trifluormethyl, Nitro, Cyano, $C_1$-$C_4$-alkoxy, $C_1$-$C_4$-Alkylthio, gelten, wobei Alkoxy und Alkylthio wiederum durch ein oder mehrere Fluor oder Phenyl substituiert sein können, für den Rest

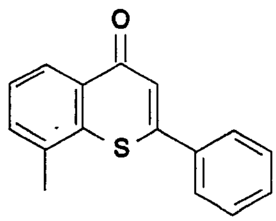

oder

für Pentafluorphenyl, Thienyl, Furyl, Pyridyl oder Benzoxadiazolyl steht, für geradkettiges oder verzweigtes, gegebenenfalls durch Pyridyl substituiertes $C_1$-$C_6$-Alkyl steht, oder für Cyclopentyl oder Cyclohexyl steht,

$R^6$ für Wasserstoff oder
für geradkettiges oder verzweigtes $C_1$-$C_3$-Alkyl steht,
$R_7$ für die Gruppe

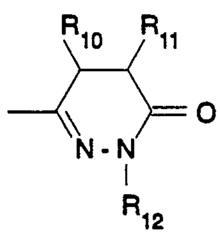 oder 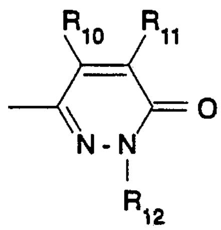

wobei

$R_{10}$, $R_{11}$ gleich oder verschieden sein können und für geradkettiges oder verzweigtes $C_1$-$C_4$-Alkyl stehen
$R_{12}$ die gleiche Bedeutung hat wie $R_6$ und wobei $R_6$ und $R_{12}$ gleich oder verschieden sein können,

in Form von Isomeren, Isomerengemischen, Racematen und optischen Antipoden sowie deren physiologisch unbedenklichen Salze.

2. Verbindungen gemäß Anspruch 1 zur Bekämpfung von Erkrankungen.

3. Verbindungen gemäß Anspruch 1, zur Behandlung von Herzinsuffizienz, von Thrombosen, von Thromboembolien, von Ischämien, zur Beeinflussung des Blutzuckerspiegels, des Kreislaufs, als Koronartherapeutikum und Antiarrhythmikum.

4. Arzneimittel enthaltend eine Verbindung gemäß dem Anspruch 1.

5. Verwendung der Verbindungen gemäß Anspruch 1, zur Herstellung von Arzneimitteln zur Bekämpfung von Erkrankungen.

6. Verwendung der Verbindungen gemäß Anspruch 1, zur Herstellung von Arzneimitteln zur Behandlung von Herzinsuffizienz, von Thrombosen, von Thromboembolien, von Ischämien, zur Beeinflussung des Blutzuckerspiegels, des Kreislaufs, als Koronartherapeutikum und Antiarrhythmikum.

7. Verfahren zur Herstellung von 1,4-Dihydropyridincarbonsäureamiden der allgemeinen Formel I

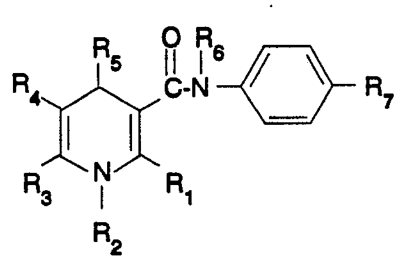

(I)

in welcher

$R_1$, $R_3$ gleich oder verschieden sind und
für Cyano,
für gegebenenfalls durch ein oder mehrere Fluor, Chlor, Brom, $C_1$-$C_2$-Alkoxycarbonyl, Acetyloxy, Pyridyl oder Hydroxy substituiertes Methyl oder Ethyl stehen,
$R_2$ für Wasserstoff, Methyl oder Ethyl steht,
$R_4$ für Wasserstoff, Nitro, Cyano oder für den Rest $COR^8$, $CO_2R^8$ oder $SO_2R^8$ steht,

wobei

$R^8$ für geradkettiges, verzweigtes oder cyclisches Alkyl oder Alkenyl mit bis zu 6 C-Atomen steht, das gegebenenfalls substituiert ist durch ein oder mehrere Fluor, Nitro, Cyano, $C_1$-$C_3$-Alkoxy, $C_1$-$C_3$-Alkylthio, Methylbenzylamino, gegebenenfalls durch Methoxy substituiertes Phenyl, Pyridyl oder Trifluormethyl oder
wobei $R^8$ eine direkte Bindung zu $R^3$ darstellt (für $R^3 \neq$ Cyano),
$R^5$ für Phenyl oder Naphthyl steht, das gegebenenfalls 1 – 2 gleiche oder verschiedene Substituenten tragen kann, wobei als Substituenten Methyl, Trifluormethyl, Nitro, Cyano, $C_1$-$C_4$-Alkoxy, $C_1$-$C_4$-Alkylthio, gelten, wobei Alkoxy und Alkylthio wiederum durch ein oder mehrere Fluor oder Phenyl substituiert sein können, für den Rest

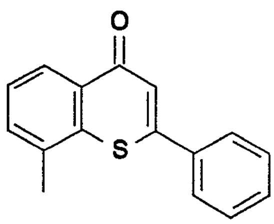

oder
für Pentafluorphenyl, Thienyl, Furyl, Pyridyl oder Benzoxadiazolyl steht,
für geradkettiges oder verzweigtes, gegebenenfalls durch Pyridyl substituiertes $C_1$-$C_6$-Alkyl steht, oder für Cyclopentyl oder Cyclohexyl steht,
$R^6$ für Wasserstoff oder
für geradkettiges oder verzweigtes $C_1$-$C_3$-Alkyl steht,
$R_7$ für die Gruppe

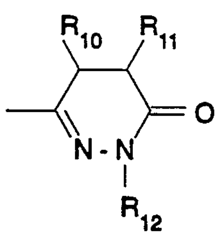 oder 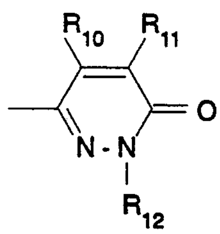

wobei

$R_{10}$, $R_{11}$ gleich oder verschieden sein können und
für geradkettiges oder verzweigtes $C_1$-$C_4$-Alkyl stehen
$R_{12}$ die gleiche Bedeutung hat wie $R_6$ und wobei und $R_{12}$ gleich oder verschieden sein können,

dadurch gekennzeichnet, daß man zur Herstellung derjenigen Verbindungen, in denen $R_8$ keine Bindung zu $R_3$ aufweist,

[A] Aldehyde der allgemeinen Formel (II)

$$R_5-\underset{\underset{\displaystyle H}{|}}{C}=O \qquad \text{(II)}$$

in welcher

$R_5$ die oben angegebene Bedeutung hat, mit Ketoverbindungen der allgemeinen Formel (III)

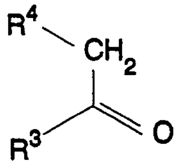

(III)

in welcher $R_3$, $R_4$ die angegebene Bedeutung haben, oder deren Kondensationsprodukte der allgemeinen Formel (IV)

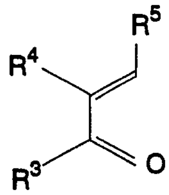

(IV)

in welcher $R_3$, $R_4$, $R_5$ die oben angegebene Bedeutung haben, mit Enaminen der allgemeinen Formel (V)

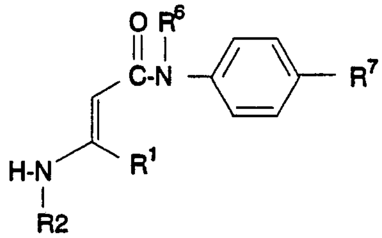

(V)

in welcher $R_1$, $R_2$, $R_6$, $R_7$ die oben angegebene Bedeutung haben, umsetzt, oder

[B] Aldehyde der allgemeinen Formel (II) und Ketoverbindungen der allgemeinen Formel (VI)

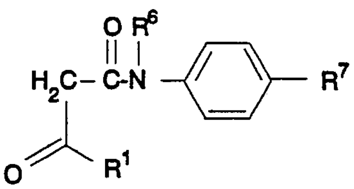

(VI)

in welcher $R_1$, $R_6$, $R_7$ die oben angegebene Bedeutung haben, oder deren Kondensationsprodukte der allgemeinen Formel (VII),

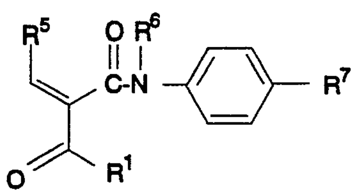

(VII)

in welcher $R_1$, $R_5$, $R_5$, $R_7$ die oben angegebene Bedeutung haben, mit Enaminen der allgemeinen Formel (VIII)

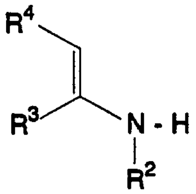

(VIII)

in welcher $R_2$, $R_3$, $R_4$ die oben angegebene Bedeutung haben, umsetzt, oder

[C] 1,3-Dihydropyridin-carbonsäuren der allgemeinen Formel (IX)

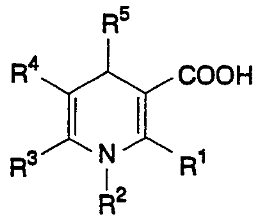

(IX)

in welcher $R_1$ – $R_5$ die oben angegebene Bedeutung haben, nach bekannten Methoden, gegebenenfalls über ein reaktives Säurederivat, mit Verbindungen der allgemeinen Formel (X),

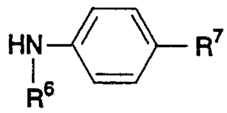

(X)

in welcher $R_6$, $R_7$ die oben angegebene Bedeutung haben, gegebenenfalls in einem inerten organischen Lösungsmittel umsetzt,
oder zur Herstellung derjenigen Verbindungen, in denen $R_4$ für die Gruppe $CO_2R_8$ steht und $R_8$ eine Bindung zu $R_3$ aufweist,

[D] Verbindungen der allgemeinen Formel Ia

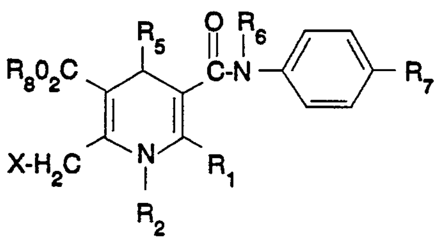

(Ia)

in welcher

| | |
|---|---|
| $R_1$, $R_2$, $R_5$-$R_7$ | die oben angegebene Bedeutung, |
| $R_8$ | die oben angegebene Bedeutung hat aber keine direkte Bindung bedeutet und |
| X | für Halogen steht, |

pyrolisiert oder falls X für O-Acetyl oder O-Benzyl steht, gegebenenfalls in Anwesenheit von Basen cyclisiert.

8. Verfahren gemäß Anspruch 7, dadurch gekennzeichnet, daß man die Verfahrensvarianten A und B bei Temperaturen von 10 – 200°C durchführt.

9. Verfahren gemäß Anspruch 7, dadurch gekennzeichnet, daß man die Verfahrensvariante C bei Temperaturen von –70°C bis +60°C durchführt.

10. Verfahren gemäß Anspruch 7, dadurch gekennzeichnet, daß man bei der Verfahrensvariante D die Pyrolyse bei Temperaturen von 20 – 300°C durchführt.

**Claims**

1. 1,4-Dihydropyridinecarboxamides of the general formula I

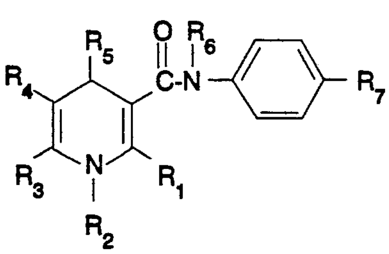

(I)

in which

$R_1$ and $R_3$ are identical or different and represent cyano, or methyl or ethyl which is optionally substituted by one or more fluorine, chlorine, bromine, $C_1$-$C_2$-alkoxycarbonyl, acetoxy, pyridyl or hydroxyl groups,
$R_2$ represents hydrogen, methyl or ethyl,
$R^4$ represents hydrogen, nitro, cyano or the radical $COR^8$, $CO_2R^8$ or $SO_2R^8$,

wherein

$R^8$ represents linear, branched or cyclic alkyl or alkenyl each of which has up to 6 C atoms and is optionally substituted by one or more fluorine, nitro, cyano, $C_1$-$C_3$-alkoxy, $C_1$-$C_3$-alkylthio, methylbenzylamino, phenyl which is optionally substituted by methoxy, or pyridyl or trifluoromethyl groups

or wherein

$R^8$ represents a direct bond to $R_3$ (in the event that $R_3$ is not cyano),
$R_5$ represents phenyl or naphthyl which can optionally carry 1 – 2 identical or different substituents, suitable substituents being methyl, trifluoromethyl, nitro, cyano or $C_1$-$C_4$-alkoxy or $C_1$-$C_4$-alkylthio, it being possible for alkoxy and alkylthio to be substituted in turn by one or more fluorine or phenyl groups, or
$R_5$ represents the radical

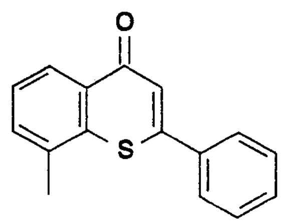

or pentafluorophenyl, thienyl, furyl, pyridyl or benzoxadiazolyl or linear or branched $C_1$-$C_6$-alkyl which is optionally substituted by pyridyl, or represents cyclopentyl or cyclohexyl,
$R_6$ represents hydrogen or linear or branched $C_1$-$C_3$-alkyl, and
$R_7$ represents the group

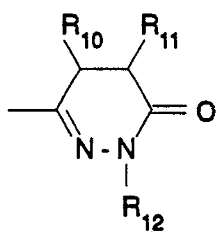

or

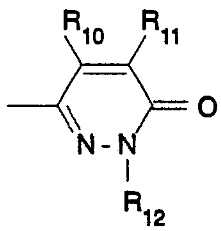

wherein

$R_{10}$ and $R_{11}$ can be identical or different and represent linear or branched $C_1$-$C_4$-alkyl and
$R_{12}$ has the same meaning as $R_6$, it being possible for $R_6$ and $R_{12}$ to be identical or different,

in the form of isomers, mixtures of isomers, racemates and optical antipodes, and physiologically acceptable salts thereof.

2. Compounds according to Claim 1 for combating diseases.

3. Compounds according to Claim 1 for the treatment of cardiac insufficiency, thromboses, thromboembolisms and ischaemias, for influencing the blood sugar level and the circulation and as a coronary therapeutic agent and anti-arrhythmic agent.

4. Medicaments containing a compound according to Claim 1.

5. The use of the compounds according to Claim 1 for the preparation of medicaments for combating diseases.

6. The use of the compounds according to Claim 1 for the preparation of medicaments for the treatment of cardiac

insufficiency, thromboses, thromboembolisms and ischaemias for influencing the blood sugar level and the circulation and as a coronary therapeutic agent and anti-arrhythmic agent.

7. Process for the preparation of 1,4-dihydropyridinecarboxamides of the general formula I

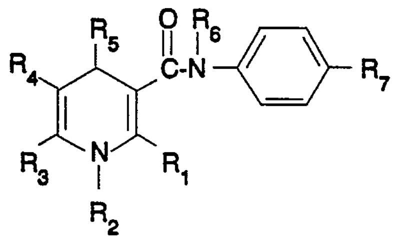

(I)

in which

| | |
|---|---|
| $R_1$ and $R_3$ | are identical or different and represent cyano, or methyl or ethyl which is optionally substituted by one or more fluorine, chlorine, bromine, $C_1$-$C_2$-alkoxycarbonyl, acetoxy, pyridyl or hydroxyl groups, |
| $R_2$ | represents hydrogen, methyl or ethyl, |
| $R_4$ | represents hydrogen, nitro, cyano or the radical $COR^8$, $CO_2R^8$ or $SO_2R^8$, |

wherein

$R^8$ represents linear, branched or cyclic alkyl or alkenyl each of which has up to 6 C atoms and is optionally substituted by one or more fluorine, nitro, cyano, $C_1$-$C_3$-alkoxy, $C_1$-$C_3$-alkylthio, methylbenzylamino, phenyl which is optionally substituted by methoxy, or pyridyl or trifluoromethyl groups

or wherein

$R^8$ represents a direct bond to $R_3$ (in the event that $R_3$ is not cyano),
$R_5$ represents phenyl or naphthyl which can optionally carry 1 – 2 identical or different substituents, suitable substituents being methyl, trifluoromethyl, nitro, cyano or $C_1$-$C_4$-alkoxy or $C_1$-$C_4$-alkylthio, it being possible for alkoxy and alkylthio to be substituted in turn by one or more fluorine or phenyl groups, or
$R_5$ represents the radical

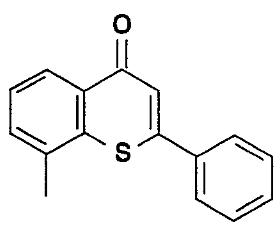

or pentafluorophenyl, thienyl, furyl, pyridyl or benzoxadiazolyl or linear or branched $C_1$-$C_6$-alkyl which is optionally substituted by pyridyl, or represents cyclopentyl or cyclohexyl,
$R_6$ represents hydrogen or linear or branched $C_1$-$C_3$-alkyl, and
$R_7$ represents the group

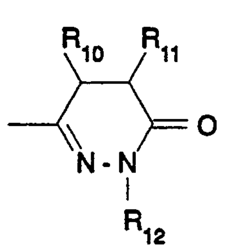

or

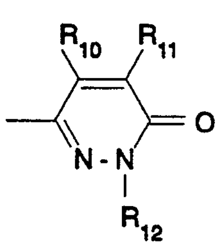

wherein

| | |
|---|---|
| $R_{10}$ and $R_{11}$ | can be identical or different and represent linear or branched $C_1$-$C_4$-alkyl and |
| $R_{12}$ | has the same meaning as $R_6$, it being possible for $R_6$ and $R_{12}$ to be identical or different, |

characterised in that, in order to prepare those compounds in which $R_8$ does not contain a bond to $R_3$,

[A] aldehydes of the general formula (II)

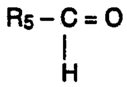

(II)

in which

$R_5$ has the meaning indicated above, are reacted with keto compounds of the general formula (III)

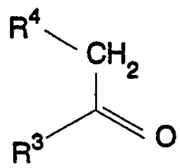

(III)

in which

$R_3$ and $R_4$ have the meaning indicated, or condensation products thereof of the general formula (IV)

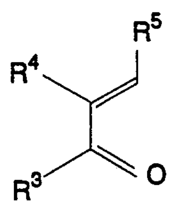

(IV)

in which

$R_3$, $R_4$ and $R_5$ have the meaning indicated above, are reacted with enamines of the general formula (V)

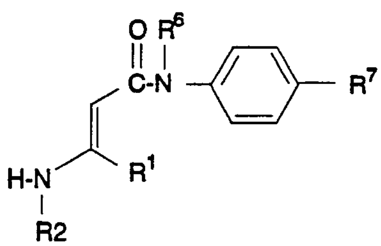

(V)

in which

$R_1$, $R_2$, $R_6$ and $R_7$ have the meaning indicated above,

or

[B] aldehydes of the general formula (II) and keto compounds of the general formula (VI)

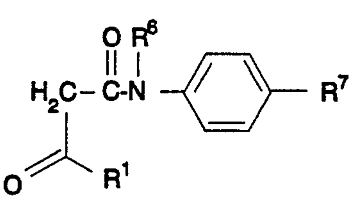

(VI)

in which

$R_1$, $R_6$ and $R_7$ have the meaning indicated above, or condensation products thereof of the general formula (VII)

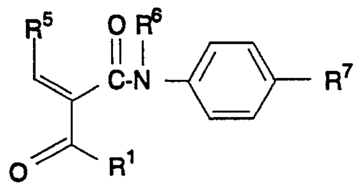

(VII)

in which

$R_1$, $R_5$, $R_6$ and $R_7$ have the meaning indicated above, are reacted with enamines of the general formula (VIII)

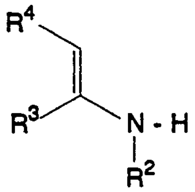

(VIII)

in which

$R_2$, $R_3$ and $R_4$ have the meaning indicated above, or

[C] 1,3-dihydropyridinecarboxylic acids of the general formula (IX)

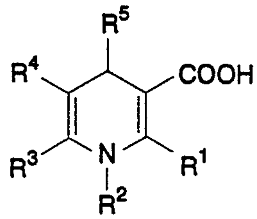

(IX)

in which

$R_1$-$R_5$ have the meaning indicated above, are reacted in accordance with known methods, if appropriate via a reactive acid derivative, with compounds of the general formula (X)

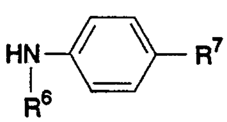

(X)

in which

$R_6$ and $R_7$ have the meaning indicated above, if appropriate in an inert organic solvent, or, in order to prepare those compounds in which $R_4$ represents the group $CO_2R_8$ and $R_8$ contains a bond to $R_3$,

[D] compounds of the general formula Ia

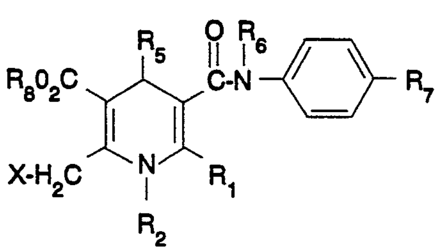

(Ia)

in which

$R_1$, $R_2$ and $R_5$–$R_7$ have the meaning indicated above,
$R_8$ has the meaning indicated above but does not denote a direct bond and
X represents halogen,

are pyrolysed or, if X represents O-acetyl or O-benzyl, are, if appropriate, cyclised in the presence of bases.

8. Process according to Claim 7, characterised in that the process variants A and B are carried out at temperatures of 10 – 200°C.

9. Process according to Claim 7, characterised in that the process variant C is carried out at temperatures of –70°C to +60°C.

10. Process according to Claim 7, characterised in that, in the process variant D, the pyrolysis is carried out at temperatures of 20 – 300°C.

**Revendications**

1. Amides de l'acide 1,4-dihydropyridine-carboxylique de formule générale (I)

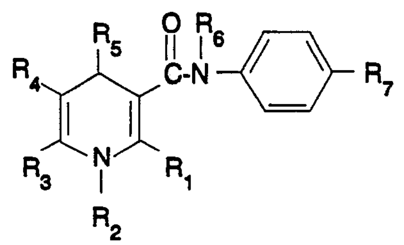

(I)

dans laquelle

$R_1$, $R_3$ sont identiques ou différents et représentent des groupes cyanos, des groupes méthyles ou éthyles éventuellement substitués par un ou plusieurs fluor, chlore, brome, alcoxycarbonyl en $C_1$-$C_2$, acétyloxy, pyridyle ou hydroxy,
$R_2$ représente l'hydrogène, un groupe méthyle ou éthyle,
$R_4$ représente l'hydrogène, un groupe nitro, cyano ou le reste $COR^8$, $CO_2R^8$ ou $SO_2R^8$,
$R^8$ étant un groupe alkyle ou alcényle linéaire, ramifié ou cyclique ayant jusqu'à 6 atomes de C, qui est éventuellement substitué par un ou plusieurs fluor, nitro, cyano, alcoxy en $C_1$-$C_3$, alkylthio en $C_1$-$C_3$, méthylbenzylamino, phényle éventuellement substitué par méthoxy, pyridyle ou trifluorométhyle, où
$R^8$ représentant un liaison directe avec $R^3$ (pour $R^3$ différent de cyano),
$R^5$ représente un groupe phényle ou un naphtyle, qui peut éventuellement porter 1 ou 2 substituants identiques ou différents, les substituants pouvant être des groupes méthyle, trifluorométhyle, nitro, cyano, alcoxy en $C_1$-$C_4$, alkylthio en $C_1$-$C_4$, les groupes alcoxy et alkylthio pouvant être à leur tour substitués par un ou plusieurs fluors ou phényles,
représente le reste

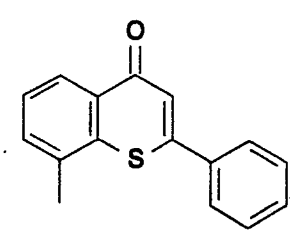

ou

représente un groupe pentafluorophényle, thiényle, furyle, pyridyle ou benzoxadiazolyle,
représente un groupe alkyle en $C_1$-$C_6$ linéaire ou ramifié, éventuellement substitué par pyridyle, ou
représente un groupe cyclopentyle ou cyclohexyle,
$R^6$ représente l'hydrogène ou un groupe alkyle en $C_1$-$C_3$ linéaire ou ramifié,
$R_7$ représente le groupe

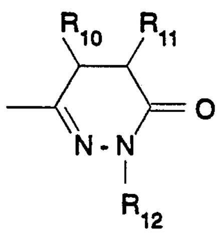

ou

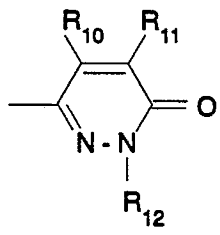

dans lequel

$R_{10}$, $R_{11}$ peuvent être identiques ou différents et représentent un groupe alkyle en $C_1$-$C_4$ linéaire ou ramifié,
$R_{12}$ a la même signification que $R_6$ et $R_6$ et $R_{12}$ peuvent être identiques ou différents,
sous forme d'isomères, de mélanges d'isomères, de racémates et d'antipodes optiques de même que de leurs sels qui ne présentent pas d'inconvénients du point de vue physiologique.

2. Composés selon la revendication 1 pour combattre les maladies.

3. Composés selon la revendication 1 pour le traitement des insuffisances cardiaques, des thromboses, des thromboembolies, des ischémies, pour influencer la teneur en sucre du sang, la circulation, en tant qu'agents thérapeutiques coronaires et qu'agents anti-arythmie.

4. Médicament contenant un composé selon la revendication 1.

5. Utilisation des composés selon la revendication 1 pour la préparation de médicaments pour combattre les maladies.

6. Utilisation des composés selon la revendication 1 pour la préparation de médicaments pour le traitement des insuffisances cardiaques, des thromboses, des thromboembolies, des ischémies, pour influencer la teneur en sucre du sang, la circulation, en tant qu'agents thérapeutiques coronaires et qu'agents anti-arythmie.

7. Procédé de préparation des amides de l'acide 1,4-dihydropyridine carboxylique de formule générale (I):

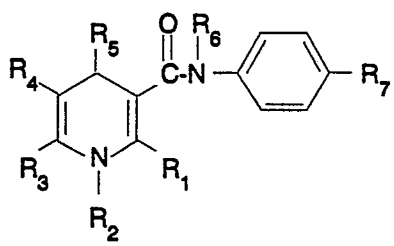

(I)

dans laquelle

$R_1$, $R_3$ sont identiques ou différents et représentent des groupes cyano, des groupes méthyles ou éthyles éventuellement substitués par ou plusieurs fluor, chlore, brome, alcoxycarbonyle en $C_1$-$C_2$, acétyloxy, pyridyle ou hydroxy,
$R_2$ représente l'hydrogène, un groupe méthyle ou éthyle,
$R_4$ représente l'hydrogène, un groupe nitro, cyano ou le reste $COR^8$, $CO_2R^8$ ou $SO_2R^8$,
$R^8$ étant un groupe alkyle ou alcényle linéaire, ramifié ou cyclique ayant jusqu'à 6 atomes de C, qui est éventuellement substitué par un ou plusieurs fluor, nitro, cyano, alcoxy en $C_1$-$C_3$, alkylthio en $C_1$-$C_3$, méthylbenzylamino, phényle éventuellement substitué par méthoxy, pyridyle ou trifluorométhyle, ou
$R^8$ représentant une liaison directe avec $R^3$ (pour $R^3$ différent de cyano),
$R^5$ représente un groupe phényle ou naphtyle oui peut éventuellement porter un ou deux substituants identiques ou différents, les substituants pouvant être des groupes méthyle, trifluorométhyle, nitro, cyano, alcoxy en $C_1$-$C_4$, alkylthio en $C_1$-$C_4$, les groupes alcoxy et alkylthio pouvant être à leur tour substitués par un ou plusieurs fluors ou phényles,

le reste

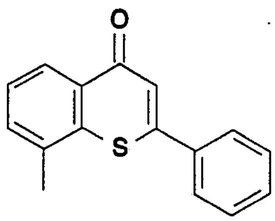

ou

un groupe pentafluorophényle, thiényle, furyle, pyridyle ou benzoxadiazolyle,
un groupe alkyle en $C_1$-$C_6$ linéaire ou ramifié, éventuellement substitué par pyridyle, ou
un cyclopentyle ou cyclohexyle,

$R^6$ représente l'hydrogène ou un groupe alkyle en $C_1$-$C_3$ linéaire ou ramifié,

$R_7$ représente le groupe

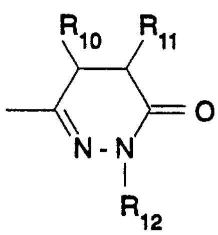

ou

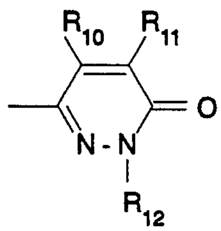

dans lequel

$R_{10}$, $R_{11}$ peuvent être identiques ou différents et représentent un groupe alkyle en $C_1$-$C_4$ linéaire ou ramifié

$R_{12}$ a la même signification que $R_6$ et $R_6$ et $R_{12}$ peuvent être identiques ou différents,

caractérisé en ce que, pour la préparation des composés dans lesquels $R_8$ ne présente pas de liaison avec $R_3$, on fait réagir

[A] des aldéhydes de formule générale (II):

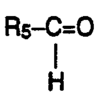

(II)

dans laquelle

$R^5$ a la signification indiquée ci-dessus,
avec des composés cétoniques de formule générale (III):

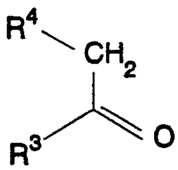

(III)

dans laquelle

$R^3$, $R^4$ ont la signification indiquée,
où leurs produits de condensation de formule générale (IV):

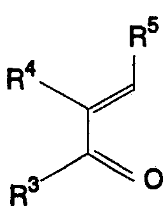

(IV)

dans laquelle

$R^3$, $R^4$, $R^5$ ont la signification donnée ci-dessus, avec des enamines de formule générale (V):

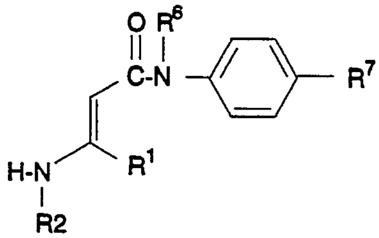

(V)

dans laquelle

$R^1$, $R^2$, $R^6$, $R^7$ ont la signification donnée ci-dessus, ou

[B] les aldéhydes de formule générale (II) avec des composés cétoniques de formule générale (VI):

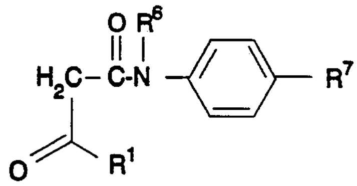

(VI)

dans laquelle

$R^1$, $R^6$, $R^7$ ont la signification donnée ci-dessus, ou leurs produits de condensation de formule générale (VII),

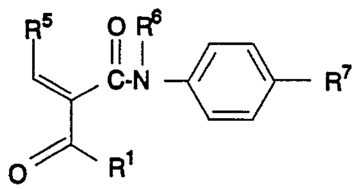

(VII)

dans laquelle

$R^1$, $R^5$, $R^6$, $R^7$ ont la signification donnée ci-dessus, avec les enamines de formule générale (VIII):

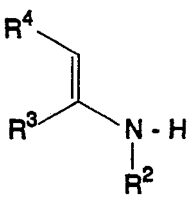

(VIII)

dans laquelle

$R^2$, $R^3$, $R^4$ ont la signification donnée ci-dessus, ou

[C] les acides 1,3-dihydropyridine carboxyliques de formule générale (IX):

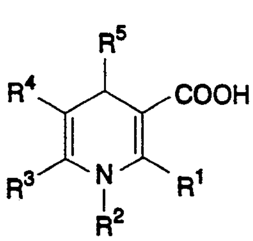

(IX)

dans laquelle

$R^1$-$R^5$ ont la signification donnée ci-dessus, selon les procédés connus, éventuellement par l'intermédiaire d'un dérivé d'acide réactif, avec des composés de formule générale (X),

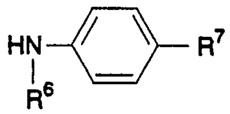

(X)

dans laquelle

$R^6$, $R^7$ ont la signification donnée ci-dessus, éventuellement dans un solvant organique inerte, ou, pour la préparation des composés dans lesquels $R_4$ représente le groupe $CO_2R_8$ et $R_8$ présente une liaison avec $R_3$, on pyrolyse

[D] les composés de formule générale Ia:

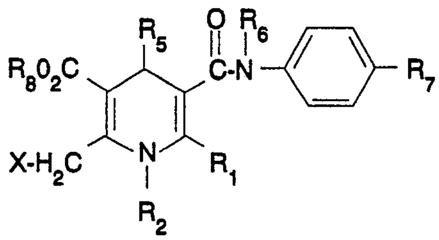

(Ia)

dans laquelle

| | |
|---|---|
| $R_1$, $R_2$, $R_5$–$R_7$ | ont la signification donnée ci-dessus, |
| $R_8$ | a la signification donnée ci-dessus mais ne représente pas de liaison directe et |
| X | représente un halogène, |

ou, au cas où X représente un groupe O-acétyle ou O-benzyle, on les cyclise éventuellement en présence de bases.

8. Procédé selon la revendication 7, caractérisé en ce que l'on réalise les variantes de procédé A et B à des températures de 10 – 200°C.

9. Procédé selon la revendication 7, caractérisé en ce que l'on réalise la variante de procédé C à des températures de –70°C à +60°C.

10. Procédé selon la revendication 7, caractérisé en ce que, dans la variante de procédé D, on réalise la pyrolyse à des températures de 20 – 300°C.